# EUROPEAN PATENT APPLICATION

(11) **EP 4 173 669 A1**
(43) Date of publication of application: **03.05.2023**
(21) Application number: 21205749.1
(22) Date of filing: 01.11.2021
(51) Int. Cl.: A61N 1/05, A61N 1/375, A61N 1/372

(54) **ANCHORING DEVICE FOR ANCHORING AN IMPLANTABLE MEDICAL DEVICE ON TISSUE**

(71) Applicant: BIOTRONIK SE & Co. KG, 12359 Berlin (DE)
(72) Inventor: KLAUS, Sebastian, 8424 Embrach (CH); HENSCHEL, Martin, 13125 Berlin (DE); HUGHES, Devan, Tualatin, 97026 (US); MUESSIG, Dirk, West Linn, 97068 (US); LANG, Volker, 12161 Berlin (DE)
(74) Representative: Biotronik Corporate Services SE

(57) **Abstract**

An anchoring device (2) for anchoring an implantable medical device (1) on tissue, comprises a housing portion (101; 133), at least one elastically deformable abutment member (22, 22A, 22B) configured to abut with tissue and extending from said housing portion (101; 133) along a length of extension (L), and a fixation arrangement (23) arranged on the at least one abutment member (22, 22A, 22B) to fix the at least on abutment member (22, 22A, 22B) on tissue over at least a portion of said length of extension (L).

## Description

The instant invention generally relates to an anchoring device for anchoring an implantable medical device on tissue and a method for anchoring an implantable medical device on tissue.

An implantable medical device of this kind may for example be an active implantable medical device (in short AIMD) which comprises electronic circuitry and an energy supply for performing a medical function within a patient's heart, such as a leadless pacemaker for implantation into the heart, for example in the right atrium of the heart.

In recent years, leadless pacemakers have received increasing attention. Leadless pacemakers, in contrast to pacemakers implanted subcutaneously using leads extending transvenously into the heart, avoid leads in that the pacemaker device itself is implanted into the heart, the pacemaker having the shape of a generally cylindrical capsule for implantation into cardiac tissue. Such leadless pacemakers exhibit the inherent advantage of not using leads, which can reduce risks for the patient involved with leads transvenously accessing the heart, such as the risk of pneumothorax, lead dislodgement, cardiac perforation, venous thrombosis and the like.

When placing an implantable medical device such as a leadless pacemaker device in the human heart, one challenge lies in designing an anchoring device allowing a fixation of the implantable medical device on cardiac tissue such that the implantable medical device is securely received and held on cardiac tissue. For this, the anchoring device on the one hand shall provide for a secure fastening of the implantable medical device on the cardiac tissue, on the other hand however shall avoid a damaging of cardiac tissue and structures within the cardiac tissue.

A typical anchoring device comprises anchoring members in the shape of tines which, when placing the implantable medical device on cardiac tissue, pierce the cardiac tissue and in this way provide for an anchoring of the implantable medical device on the cardiac tissue. For placing the implantable medical device on cardiac tissue, the implantable medical device for example is received within a sheathing device of a delivery catheter, the delivery catheter being used to access the human heart and to deliver the implantable medical device towards a location of interest by removing the sheathing device and by in this way placing the implantable medical device on cardiac tissue at the location of interest. Upon delivering the implantable medical device from the sheathing device the anchoring members engage with cardiac tissue to provide for a fastening of the implantable medical device on the cardiac tissue.

US 9,844,659 describes an assembly of an implantable medical device comprising an anchoring device having a set of active fixation tines. The active fixation tines in the set are deployable from a spring-loaded position in which distal ends of the fixation tines point away from the implantable medical device to a hooked position in which the active fixation tines bend back towards the implantable medical device.

US 9,526,891 discloses an implantable pacemaker system including a housing having a proximal end and a distal end, an arrangement of anchoring members being placed on the distal end of the housing for providing for a fastening of the implantable pacemaker system on cardiac tissue.

US 8,700,181 discloses a leadless intra-cardiac medical device configured to be implanted entirely within a heart of a patient, the device including a housing configured to be securely attached to an interior wall portion of a chamber of the heart, and a stabilizing intra-cardiac device extension connected to the housing. The stabilizing intra-cardiac device extension may include a stabilizer arm or an appendage arm, or an elongated body or a loop member configured to be passively secured within the heart.

There is a general desire for an anchoring device which allows for an easy and reliable anchoring of an implantable medical device on tissue, in particular within the ventricle or the atrium of the heart. There herein is the wish that the anchoring shall be flexibly possible at different locations.

It is an objective of the instant disclosure to provide an anchoring device for anchoring an implantable medical device on tissue and a method for anchoring an implantable medical device which allow for an easy and reliable anchoring of the implantable medical device on tissue, particularly within a patient's heart.

This objective is attained by an anchoring device comprising the features of claim 1.

In one aspect, an anchoring device for anchoring an implantable medical device on tissue comprises a housing portion, at least one elastically deformable abutment member configured to abut with tissue and extending from said housing portion along a length of extension, and a fixation arrangement arranged on the at least one abutment member to fix the at least one abutment member on tissue over at least a portion of said length of extension.

The anchoring device serves to provide a fixation of an implantable medical device on tissue, particularly within a chamber of a patient's heart, such as within the right or left ventricle or within the right or left atrium. For this, the anchoring device comprises one or multiple elastically deformable abutment members which are configured and shaped to abut with tissue such that, in an implanted state, the one or multiple abutment members with at least a portion of their length are in abutment with tissue. Because the abutment members are elastically deformable, they may adapt to a tissue geometry at a location of interest at which the anchoring device is placed, such that the abutment members may rest on tissue and may make contact with tissue over at least a portion of their length.

In one embodiment, the at least one abutment member shall not engage with tissue by piercing into tissue but shall rest on tissue to provide for an abutment and a contact with the tissue.

To establish a fixation of the one or the multiple abutment members with tissue, a fixation arrangement is provided which is configured to establish a fixation of the one or the multiple abutment members on tissue over at least a portion of the length of extension of the one or multiple abutment members. This is understood to mean that the fixation arrangement does not provide for a fixation at a single point, but over an axial range within the length of extension of the at least one deformable abutment member. The one or the multiple abutment members hence are fixed to tissue by means of the fixation arrangement over at least a portion of their length of extension, such that a fixation of the anchoring device on tissue is established.

Whereas the at least one elastically deformable abutment member shall rest on tissue such that the at least one deformable abutment member is in contact with the tissue, a fixation is provided by means of the fixation arrangement. Whereas the at least one elastically deformable abutment member may not pierce into tissue, features of the fixation arrangement, which for example may have a micro-shape, such as micro-hooks, may be in engaging contact with tissue and hence may establish a fixation of the at least one elastically deformable abutment member on tissue.

Because the at least one elastically deformable abutment member does not pierce into tissue, a damaging of tissue may at least be reduced. Because the fixation arrangement provides for a fixation over a length portion and hence over a spatially distributed area, the fixation may be established by micro-features causing only micro-damages on tissue, hence overall reducing an impact on tissue for providing for a secure and reliable, enduring fixation of the anchoring device on tissue.

In one embodiment, the at least one abutment member is connected at a first end to said housing portion and comprises a second, free end remote from the housing portion, the at least one abutment member extending over said length of extension between the first end and the second, free end. The at least one abutment member herein may have the shape of an umbrella-like shield, a wing or a linear, flexible bar, wherein one end is connected to the housing portion and another, second end is free from the housing such that the at least one abutment member is elastically deformable with respect to the housing portion.

In one embodiment, the fixation arrangement comprises a multiplicity of engaging elements distributed on the at least one abutment member across at least a portion of said length of extension, the engaging elements being configured to engage with tissue. The fixation arrangement particularly may comprise a large number of engaging elements, for example more than 10, preferably more than 50, more preferably more than 100 engaging elements which are distributed across the one or the multiple abutment members. This allows to form the engaging elements as micro-features, for example in the shape of micro-hooks, micro-spikes, micro-tines, micro-barbs, or the like, such that each engaging element only micro-punctures into tissue and provides for a limited damaging on tissue. Due to the spatial distribution of the engaging elements a secure and reliable fixation may be provided to anchor the implantable medical device on tissue. In one embodiment, the fixation arrangement comprises a plurality of abutment member, wherein at least one abutment member of the plurality of abutment members comprise the above-mentioned multiplicity of engaging element. In one embodiment, each of the plurality of abutment members comprises the above-mentioned multiplicity of engaging elements.

In another embodiment, the fixation arrangement comprises a glue arrangement distributed on the at least one abutment member across at least a portion of said length of extension, the glue arrangement being configured to establish a gluing connection with tissue. The glue arrangement for example may be formed by a layer of glue covering at least a portion of the at least one abutment member on a side facing towards tissue. Alternatively, one or multiple glue spots, such as glue pockets, may be formed on the at least one abutment member for providing for a fixation with tissue, the glue spots being distributed over the one or multiple abutment members such that a spatially distributed fixation is established. Suitable glues include without being restricted to acrylic copolymer adhesives, starch or a spider-web adhesive or spider silk. In one embodiment, the fixation arrangement comprises a plurality of abutment member, wherein at least one abutment member of the plurality of abutment members comprise the above-mentioned glue arrangement distributed on the at least one abutment member across at least a portion of said length of extension. In one embodiment, each of the plurality of abutment members comprises the above-mentioned glue arrangement distributed on each of the abutment members across at least a portion of said length of extension.

In an initial, pre-anchoring state the glue arrangement may be covered with a liner, the liner being removed in the course of placing the anchoring device on tissue such that the glue arrangement may come into contact and may establish a gluing connection with tissue upon placing the anchoring device on tissue. As an alternative, the glue arrangement may be, an initial pre-anchoring state, covered with are formed by pre-polymers configured to act as or becoming an adhesive by being exposed to the local embodiment after deployment of the medical device. Such pre-polymers may be based on polyethylene glycol.

In one embodiment, the at least one abutment member comprises at least one elastically deformable strut. In a beneficial embodiment, the at least one abutment member comprises a multiplicity of struts which are arranged on the housing portion in a spatially distributed manner. For example, if the housing portion is to be placed on tissue in a placement direction (corresponding for example to a distal direction when advancing a catheter towards a location of interest), the multiple struts may be distributed on the housing portion along a circumferential direction about the placement direction, the abutment members hence extending from the housing portion for example in different radial directions such that a fixation of the anchoring device on tissue around the housing portion may be established.

The abutment members may simply be formed by struts extending from the housing portion, the struts being, in one embodiment, not interconnected or, in another embodiment, being interconnected for example by additional interconnecting struts.

In another embodiment, the at least one abutment member comprises a flexible sheet arrangement, for example made of a fabric, such as a woven fabric, a mesh, or a membrane, the flexible sheet arrangement for example being spanned by and extending in between an arrangement of struts connected to the housing portion.

In one embodiment, the abutment member may have an umbrella-like configuration, the abutment member being formed by an arrangement of struts across which a circumferentially closed sheet arrangement is spanned.

In another embodiment, the at least one abutment member may assume a wing-like configuration, one or multiple wing members for example being formed by an arrangement of struts and a sheet arrangement spanning across the struts.

The one or the multiple struts may for example be formed from a super-elastic metal material, such as a shape memory alloy, for example a nickel titanium alloy, for example Nitinol. Alternatively, the one or the multiple struts may be formed from a biocompatible plastics or silicone material, for example a polymer material, or a composite material.

The sheet arrangement may be formed for example from a woven fabric or mesh and may be permeable. Alternatively, the sheet arrangement may be formed from a membrane and may for example be non-permeable.

The sheet arrangement may for example be formed from a biocompatible plastics or silicone material, for example a polymer material. If the sheet arrangement for example is formed by a mesh, the sheet arrangement may as well be formed from a metal wire arrangement, for example an arrangement of wires made from a shape memory alloy, such as a nickel titanium alloy, for example Nitinol.

The fixation arrangement, including for example a multiplicity of engaging elements or a glue arrangement, may be formed on one or multiple struts. Alternatively or in addition, the fixation arrangement may be formed on a sheet arrangement of an abutment member.

The implantable medical device for example may be an active implantable medical device and for this may comprise electronic circuitry as well as an energy supply such that the implantable medical device, once implanted into the heart, may perform a control and/or monitoring function within the heart, such as a pacing function and/or a sensing function. The implantable medical device in particular may be a leadless pacemaker device configured for implantation into an atrium of the patient's heart, in particular the right atrium of the patient's heart.

In one embodiment, the implantable medical device comprises an electrode configured to act as a pacing and/or sensing electrode, particularly a tip electrode, which may be arranged on a housing of the implantable medical device, or which may be arranged on a lead connected to the implantable medical device, the lead extending from the housing of the implantable medical device to rest on tissue. The skilled person will appreciate that the implantable medical device also comprises a second electrode acting as return electrode for the above-mentioned electrode acting as pacing and/or sensing electrode. Such return electrode may, for example, formed by at least a part of a conductive housing of the implantable medical device. The implantable medical device may particularly have the shape of a longitudinal capsule having a distal end and a proximal end. The housing herein encapsulates electric and electronic components of the device in a fluid-tight manner, an electrode being placed for example distally on the housing, wherein on the proximal end the housing may for example comprise a coupling device which may be used to establish a coupling to a delivery catheter or the like. The electrode serves to transmit (acting as pacing electrode) and/or receive (acting as sensing electrode) electrical signals to and from the cardiac tissue and for this, when the medical device is implanted into the heart, is in abutment with cardiac tissue.

In one embodiment, the implantable medical device is fixedly and non-releasably connected to the anchoring device, such that the implantable medical device is implanted together with the anchoring device and is anchored on tissue using the anchoring device. In certain embodiments, the housing portion of the anchoring device may for example be integrally formed with the housing of the implantable medical device, such that the anchoring device is fixedly arranged on the housing of the implantable medical device.

In another embodiment, the implantable medical device is to be attached to the anchoring device and may be releasably connected to the anchoring device. In certain embodiments, the anchoring device may for example be implanted first without the implantable medical device, wherein the implantable medical device is implanted in a second step and for this is placed on the anchoring device which already is anchored to tissue at a location of interest. The implantable medical device may be released from the anchoring device for example for replacing the implantable medical device.

In an embodiment in which the implantable medical device may be releasably attached to the anchoring device, the anchoring device may comprise a docking mechanism allowing for a releasable attachment of the implantable medical device to the anchoring device. The docking mechanism may be designed such that the implantable medical device may be docked onto the anchoring device when the anchoring device already is implanted in a patient, such that an implantation procedure includes the implantation of the anchoring device in a first step and the implantation of the implantable medical device by docking to the anchoring device in a second step.

In such an embodiment, the housing portion of the anchoring device is part of the docking mechanism, the housing portion allowing for a (releasable) connection of the implantable medical device to the anchoring device.

A docking mechanism of this kind may for example comprise one or multiple magnet elements which are configured to magnetically interact with the implantable medical device to connect the implantable medical device to the anchoring device. A connection of the implantable medical device to the anchoring device hence is established by magnetic attraction forces of an arrangement of magnet elements of the anchoring device and an arrangement of magnet elements of the implantable medical device. The magnet elements herein both on the side of the anchoring device and on the side of the implantable medical device may be permanent magnets, or on one side may be permanent magnets and on the other side may be a magnetically passive armatures, for example in the shape of a ferromagnetic elements which magnetically interact with permanent magnets of the respective other device.

In another embodiment, a docking mechanism may comprise a mechanical locking feature for establishing a mechanical connection in between the anchoring device and the implantable medical device, such as a positive locking connection. The mechanical locking feature may for example comprise a form-fit element for establishing a mechanical, e.g. positive locking connection in between the implantable medical device and the anchoring device.

In another embodiment, the docking mechanism may comprise a threading member into which the implantable medical device may be screwed in order to attach the implantable medical device to the anchoring device. Hence, for placing the implantable medical device on the anchoring device the implantable medical device is screwed into the threading member of the docking mechanism, wherein the connection of the implantable medical device to the anchoring device may be released by unscrewing the implantable medical device from the threading member.

In another embodiment, the docking mechanism may alternatively or in addition to the above-mentioned mechanical locking feature comprise an adhesive or glue for establishing a mechanical connection in between the anchoring device and the implantable medical device.

In one aspect, a delivery arrangement for implanting the anchoring device in a patient comprises a delivery device, generally in the shape of a delivery catheter, and an anchoring device of the kind described above. The delivery device is configured to place the anchoring device on tissue in a placement direction, wherein the delivery device comprises a receptacle for receiving the anchoring device in a delivery state. In the delivery state, herein, the at least one abutment member is elastically deformed to extend from the housing portion in a direction opposite the placement direction. Alternatively, the at least one abutment member may be bent circumferentially around the housing in a thread-like manner for delivery or in the delivery state, respectively. Such design has the advantage that larger bending radiuses and/or smaller bending angles (e.g. lower that 180°) may be realized in the delivery state of the abutment member. which may overcome physical limitation of the material of the abutment member.

In the delivery state, hence, the at least one abutment member is folded with respect to the housing portion such that the at least one abutment member is received within the receptacle of the delivery device. Once the anchoring device is caused to exit from the receptacle, the at least one abutment member folds out to come into contact with tissue, such that a fixation of the anchoring device on tissue by means of the fixation arrangement formed on the at least one abutment member may be established.

The at least one abutment member in particular may be arranged on a distal end of the housing portion, such that at least one abutment member extends proximally from the housing portion in the delivery state and folds out to abut with tissue once the anchoring device is delivered from the delivery device.

In another aspect, a method for anchoring an implantable medical device on tissue using an anchoring device is provided, the method comprising: placing a housing portion of the anchoring device on tissue; causing at least one elastically deformable abutment member extending from said housing portion along a length of extension to abut with tissue; and fixing the at least one abutment member on tissue over at least a portion of said length of extension using a fixation arrangement arranged on the at least one abutment member.

Herein, in one embodiment, said step of placing includes: placing the anchoring device on tissue using a delivery device, the anchoring device in a delivery state being received in a receptacle of the delivery device, wherein in the delivery state the at least one abutment member is elastically deformed to extend from the housing portion in a direction opposite the placement direction.

In addition, said step of causing the at least one abutment member to abut with tissue may include: exiting the anchoring device from the delivery device such that the at least one abutment member elastically folds out from the delivery state to abut with tissue.

The advantages and advantageous embodiments described above for the anchoring device equally apply also to the method, such that it shall be referred to the above in this respect.

The various features and advantages of the present invention may be more readily understood with reference to the following detailed description and the embodiments shown in the drawings. Herein,
- Fig. 1: shows a schematic view of the human heart;
- Fig. 2: shows a view of an embodiment of an implantable medical device including an anchoring device;
- Fig. 3: shows a view of another embodiment of an implantable medical device including an anchoring device;
- Fig. 4: shows a view of yet another embodiment of an implantable medical device including an anchoring device;
- Figs. 5A - 5D: show views for inserting the implantable medical device of Fig. 3 into a receptacle of a delivery device;
- Figs. 6A - 6D: show views of placing the implantable medical device on tissue for establishing an anchoring using the anchoring device;
- Fig. 7A: shows an embodiment of an implantable medical device comprising an anchoring device;
- Fig. 7B: shows an enlarged view of a portion of the anchoring device of Fig. 7A;
- Figs. 8A - 8D: show views of placing the implantable medical device on tissue for establishing an anchoring using the anchoring device, in another embodiment;
- Fig. 9: shows an embodiment of an anchoring device forming a docking mechanism;
- Fig. 10: shows a view of another embodiment of an anchoring device forming a docking mechanism;
- Fig. 11A: shows a view of arranging an implantable medical device on the anchoring device of Fig. 9;
- Fig. 11B: shows a view of the implantable medical device on the anchoring device;
- Fig. 11C: shows a view of releasing the implantable medical device from the anchoring device;
- Fig. 12A: shows a view of arranging an implantable medical device on the anchoring device of Fig. 10; and
- Fig. 12B: shows a view of the implantable medical device on the anchoring device;

Subsequently, embodiments shall be described in detail with reference to the drawings. In the drawings, like reference numerals designate like structural elements.

It is to be noted that the embodiments are not limiting for the instant disclosure, but merely represent illustrative examples.

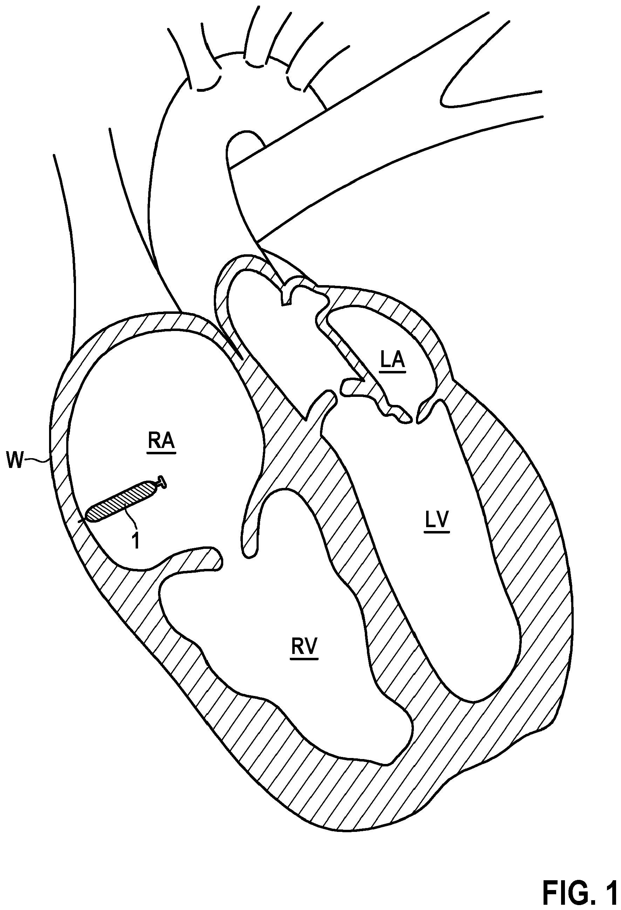

Fig. 1 shows, in a schematic drawing, a human heart comprising a right atrium RA, a right ventricle RV, a left atrium LA and a left ventricle LV, an implantable medical device 1 being implanted for example into the right atrium RA, the implantable medical device 1 e.g. having the shape of a leadless pacemaker device for providing a pacing of the heart's activity at the right atrium RA.

For implantation the implantable medical device 1 by means of a delivery system is placed in a chamber of the heart, for example in a ventricle LV, RV or an atrium LA, RA, and is delivered from the delivery system such that it comes to rest on intra-cardiac tissue, i.e. on an intra-cardiac wall W, and is fastened to intra-cardiac tissue.

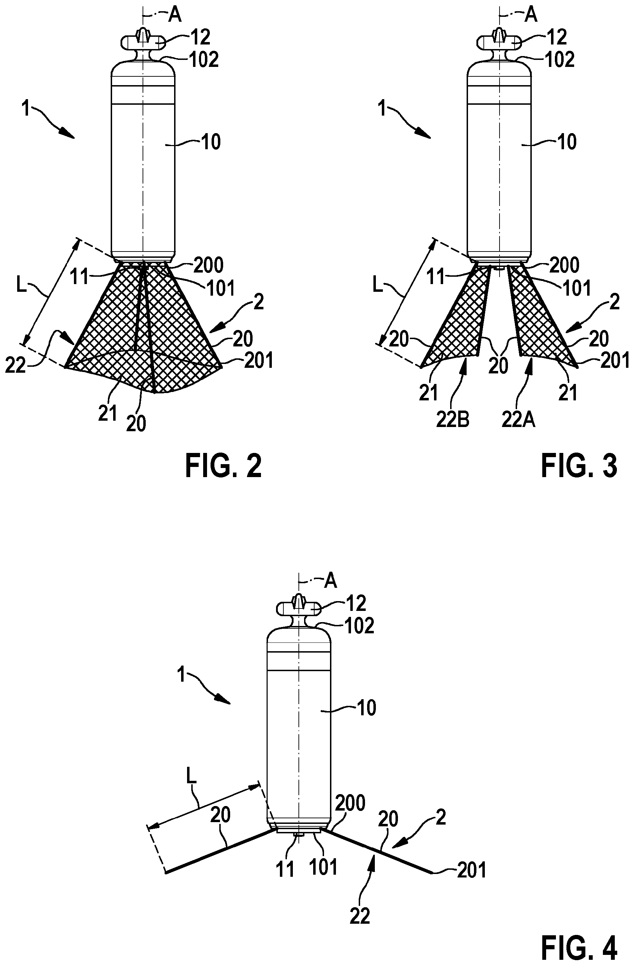

Fig. 2 shows an embodiment of an implantable medical device 1 in the shape of a leadless pacemaker device, the implantable medical device 1 having a housing 10 being configured to be placed on intra-cardiac tissue, for example an atrial wall W. The implantable medical device 1 has the shape of a capsule extending longitudinally along a longitudinal axis A, the housing 10 encapsulating electrical and electronic components of the implantable medical device 1 therein in a fluid-tight manner.

In the embodiment of Fig. 2, the implantable medical device 1 comprises an electrode 11 arranged at a distal end 101 of the housing 10 configured to act as a pacing and/or sensing electrode. The implantable medical device 1, with its distal end 101, is to be placed on tissue such that the electrode 11 comes into electrical contact with an electrical conduction system of the heart for providing an electrical stimulation within the heart, or for receiving (acting as sensing electrode) and sensing (acting as pacing electrode) signals in order to monitor a cardiac activity within the heart. The implantable medical device 1 also comprises a return electrode for the electrode 11 and may be formed by a part of the housing 10 or the coupling device 12 described below.

At a proximal end 102 of the housing 10 a coupling device 12 is formed, the coupling device 12 allowing to couple the implantable medical device 1 to a delivery device, for example a tether of a delivery device, for delivering the implantable medical device 1 towards a location of interest.

The implantable medical device 1 in the embodiment of Fig. 2 comprises an anchoring device 2 which is arranged at the distal end 101 of the housing 10, the anchoring device 2 serving to anchor the implantable medical device 1 on tissue once the implantable medical device 1 is introduced into the heart and is placed at a location of interest within the heart.

In the embodiment of Fig. 2, the anchoring device 2 comprises an arrangement of struts 20 across which a sheet arrangement 21 is spanned, the struts 20 together with the sheet arrangement 21 forming an abutment member 22 having an umbrella-like configuration for abutment with tissue at a location of interest.

The struts 20 at one end 200 of the abutment member 22 are connected to the distal end 101 of the housing 10 and linearly extend from the housing 10 towards a second, free end 201 of the abutment member 22. The sheet arrangement 21 is circumferentially closed such that it extends circumferentially across the struts 20, the sheet arrangement 21 being elastically stretchable such that the abutment member 22 may be elastically deformed for delivery using a delivery device and for abutting with tissue. Alternatively, the abutment member 22 may have a curved shape in the deployed state.

The struts 20 may for example be formed from an elastic metal material, for example a shape memory alloy, for example a nickel titanium alloy, particularly nitinol. Alternatively, the struts 20 may be formed from a biocompatible plastics or silicone material, for example a polymer material. In Fig. 2 the struts 20 are shown in a relaxed position, wherein the struts 20 may be elastically deformed from the relaxed position and may be tensioned to for example abut tissue under elastic tensioning.

The sheet arrangement 21 flatly extends in between the struts 20 and may be formed for example from a woven fabric, a mesh or a membrane. The sheet arrangement 21 may for example be made from a plastics or silicone material, or, if formed as a mesh, from a wire arrangement, for example from nitinol wires.

Referring now to Fig. 3, in another embodiment the anchoring device 2 comprises multiple abutment members 22A, 22B having a wing-like configuration, each abutment member 22A, 22B being formed from an arrangement of multiple struts, for example two struts, in between which a sheet arrangement 21 extends.

Referring now to Fig. 4, in yet another embodiment the abutment members 22 may be formed by struts 20, without an interconnecting sheet arrangement extending in between the struts 20.

In each case, the one or the multiple abutment members 22, 22A, 22B extend along a length of extension L from the distal end 101 of the housing 10, the length L being measured in between the inner end 200 of the respective abutment member 22, 22A, 22B and the free, outer end 201 of the respective abutment member 22, 22A, 22B.

The one or the multiple abutment members 22, 22A, 22B are flexibly deformable such that the anchoring device 2 with its abutment members 22, 22A, 22B may flatly abut with tissue and herein may adapt to a specific geometric shape of the tissue in the vicinity of the location of interest.

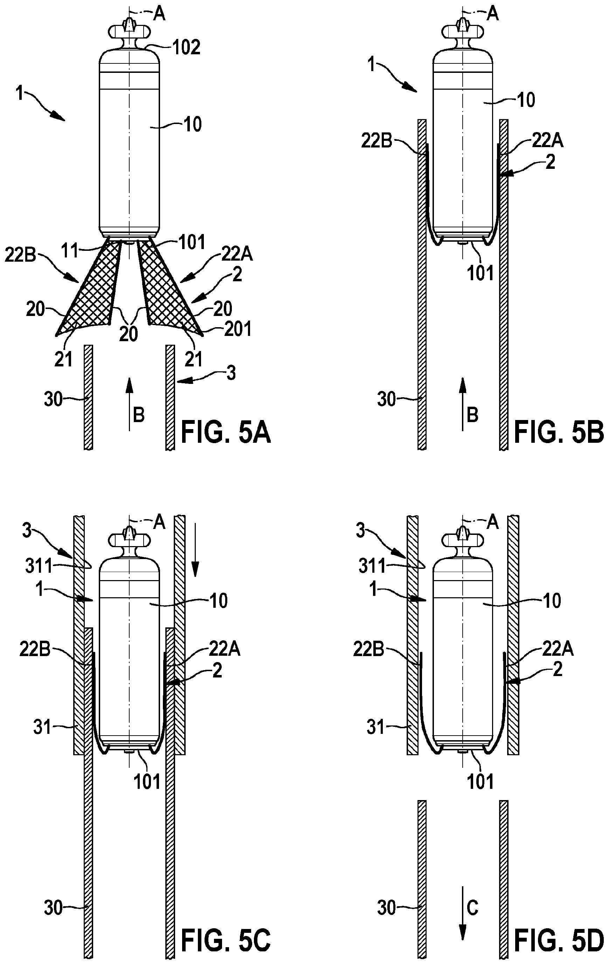

Referring now to Figs. 5A to 5D, for delivering the implantable medical device 1 with the anchoring device 2 arranged thereon the implantable medical device 1 is to be introduced into a delivery device 3, the delivery device 3 having a catheter 31 forming a receptacle 311 for receiving the implantable medical device 1 therein.

For introducing the implantable medical device 1 with the anchoring device 2 into the receptacle 311, an assembly tube 30 is placed on the implantable medical device 1 in an assembly direction B, the assembly tube 30 being slid upon the implantable medical device 1 via the distal end 101 such that the abutment members 22A, 22B (in Figs. 5A to 5D the assembly is shown by means of illustration for the embodiment of Fig. 3) are folded with respect to the housing 10 to extend in the assembly direction B from the distal end 101 alongside the housing 10 towards the proximal end 102 of the housing 10, as shown in the transition of Fig. 5A to Fig. 5B.

Once the implantable medical device 1 is received within the assembly tube 30, the assembly tube 30 is introduced into the receptacle 311 of the catheter 31, as illustrated in Fig. 5C. By removing the assembly tube 30 in a removal direction C the implantable medical device 1 comes to rest within the receptacle 311 of the catheter 31 of the delivery device 3, the implantable medical device 1 hence being ready for implantation into a patient.

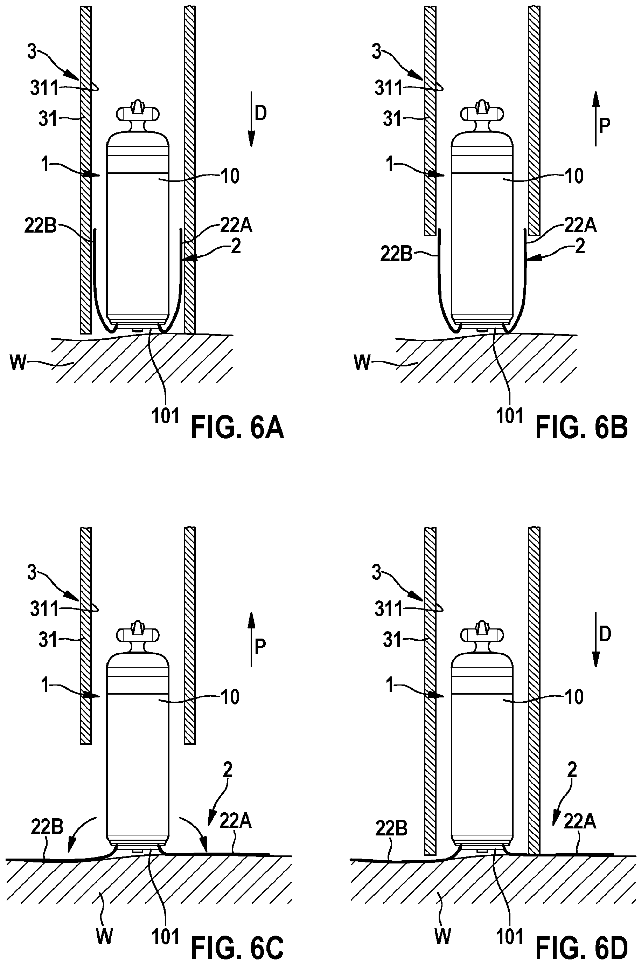

Referring now to Figs. 6A to 6D, for implanting the implantable medical device 1 the delivery device 3 with the catheter 31 is placed in a placement direction D, corresponding to a distal direction, on an intra-cardiac wall W at a location of interest such that the implantable medical device 1 with its distal end 101 comes to rest on tissue, as visible from Fig. 6A. By now withdrawing the catheter 31 in a proximal direction P while pushing out the implantable medical device 1 from the catheter 31, the implantable medical device 1 comes to rest on the intra-cardiac wall W and, once the catheter 31 is sufficiently removed to unsheathe the abutment members 22A, 22B, the abutment members 22A, 22B unfold to abut with tissue at the inside of the intra-cardiac wall W, as visible from the transition of Fig. 6B to Fig. 6C.

Once the abutment members 22A, 22B are unfolded and are in abutment with tissue, the catheter 31 of the delivery device 3 may again be moved in the placement direction D to be approached towards the abutment members 22A, 22B, such that the abutment members 22A, 22B may be pressed into abutment with tissue in order to provide for a fixation of the anchoring device 2 and, in this way, of the implantable medical device 1 on tissue.

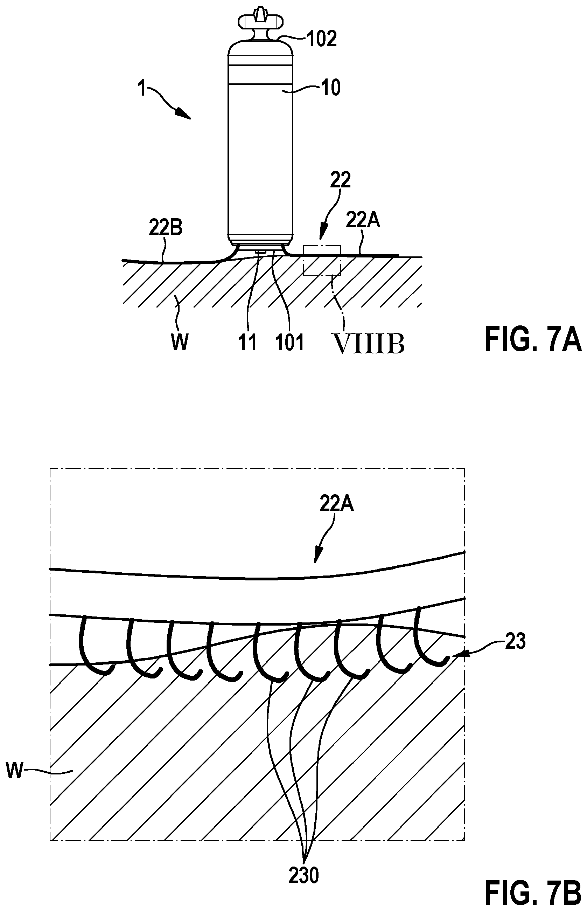

For anchoring the anchoring device 2 on tissue, the abutment members 22, 22A, 22B of the embodiments of the implantable medical device 1 according to Figs. 2 to 4 are to be brought into abutment with tissue, the abutment members 22, 22A, 22B, being shaped such that the abutment members 22, 22A, 22B atraumatically rest on tissue without piercing the tissue. As illustrated in Figs. 7A and 7B, on a side facing the tissue a fixation arrangement 23 is arranged on the abutment members 22, 22A, 22B, the fixation arrangement 23 establishing a fixation of the abutment members 22, 22A, 22B to the tissue such that the anchoring device 2 may provide for a strong, reliable fixation of the implantable medical device 1 on tissue.

The fixation arrangement 23 comprises, in one embodiment, engaging elements 230, which for example are shaped by micro-structures such as micro-hooks, micro-tines, micro-spikes, micro- barbs or the like. The engaging elements 230 in particular may be shaped such that they may come into engagement with tissue by micro-puncturing the tissue, similar to a Velcro-type connection, the engaging elements 230 hence providing for a fixation while only causing a limited damaging of tissue on a microscopic scale.

The fixation arrangement 23 is shaped such that a fixation over at least a portion of the length of extension L of the respective abutment member 22, 22A, 22B is provided. Particularly, engaging elements 230 may be distributed over at least a portion of the length L and in addition along the circumference of the respective abutment member 22, 22A, 22B, such that a spatially distributed fixation in between the respective abutment member 22, 22A, 22B on tissue may be established.

In particular, the respective abutment member 22, 22A, 22B may comprise a large number of engaging elements 230, for example more than 10, preferably more than 50, more preferably more than 100 engaging elements 230.

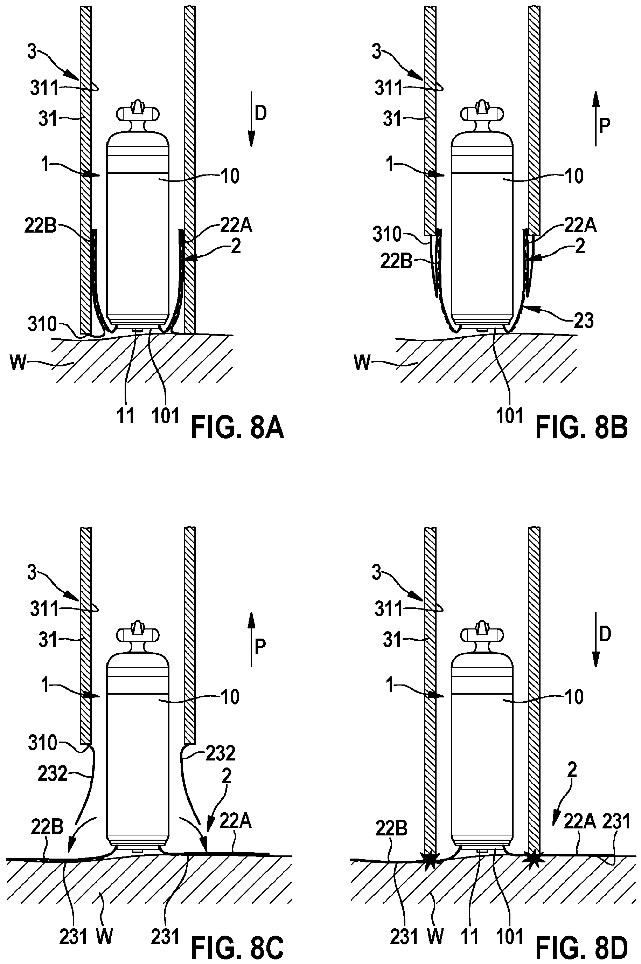

Referring now to Figs. 8A to 8D, in another embodiment the anchoring device 2 comprises a fixation arrangement 23 being formed by a glue arrangement 231, for example a glue layer, formed on the respective abutment member 22, 22A, 22B.

Herein, upon placing the implantable medical device 1 on tissue, as shown in Figs. 8A and 8B, and upon removing the catheter 31 to unsheathe the respective abutment member 22A, 22B, a liner 232 is pulled off the glue arrangement 231 of the fixation arrangement 23, such that the glue arrangement 231 comes into contact with tissue when the respective abutment member 22, 22A, 22B unfolds to abut with tissue, as shown in Fig. 8C.

For pulling the liner 232 off the glue arrangement 231, the liner 232 may for example be connected to a distal end 310 of the catheter 31, as this is shown in Fig. 8C, such that the liner 232 automatically is pulled off the respective abutment member 22, 22A, 22B when the abutment member 22, 22A, 22B folds out towards the abutment position of Fig. 8C.

Once the respective abutment member 22, 22A, 22B is unfolded, the catheter 31 may again be pushed forward in the distal placement direction D for acting onto the respective abutment member 22, 22A, 22B for pressing the glue arrangement 231 into tight contact with tissue for securing the fixation on the tissue, as illustrated in Fig. 8D.

Optionally, a curing of the glue arrangement 231, for example an ultraviolet curing, may be employed to provide for a rapid hardening of the glue arrangement 231.

In the examples of Figs. 2 to 8A-8D the anchoring device 2 is part of the implantable medical device 1, a housing portion to which the abutment members 22, 22A, 22B are connected being formed by a distal end 101 of the housing 10 of the implantable medical device 1.

In other embodiments, the anchoring device 2 however may be separate from the implantable medical device 1, the anchoring device 2 providing for a docking station to anchor the implantable medical device 1 on tissue.

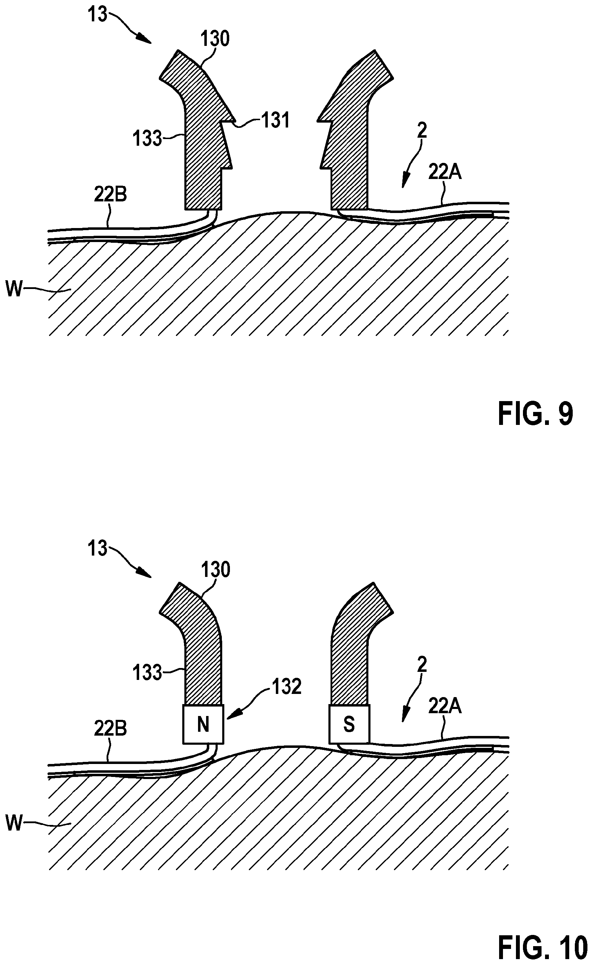

Referring now to Fig. 9, in one embodiment the anchoring device 2 may comprise a docking mechanism 13 having a housing portion 133 in the shape of a generally cylindrical bushing, the housing portion 133 forming a receptacle 130 for receiving the implantable medical device 1 therein. Within the receptacle 130, a mechanical locking feature 131 is formed, the mechanical locking feature being designed to establish a positive locking connection to the implantable medical device 1.

In another embodiment, shown in Fig. 10, the anchoring device 2 comprises a magnet arrangement 132, in addition to or instead of the mechanical locking feature 131 of Fig. 9, to establish a connection to an implantable medical device 1.

Alternatively or in addition to the mechanical locking feature 131 of Fig. 9, the anchoring device may comprise an adhesive or glue (arrangement) inside the anchoring device to establish a connection to an implantable medical device 1.

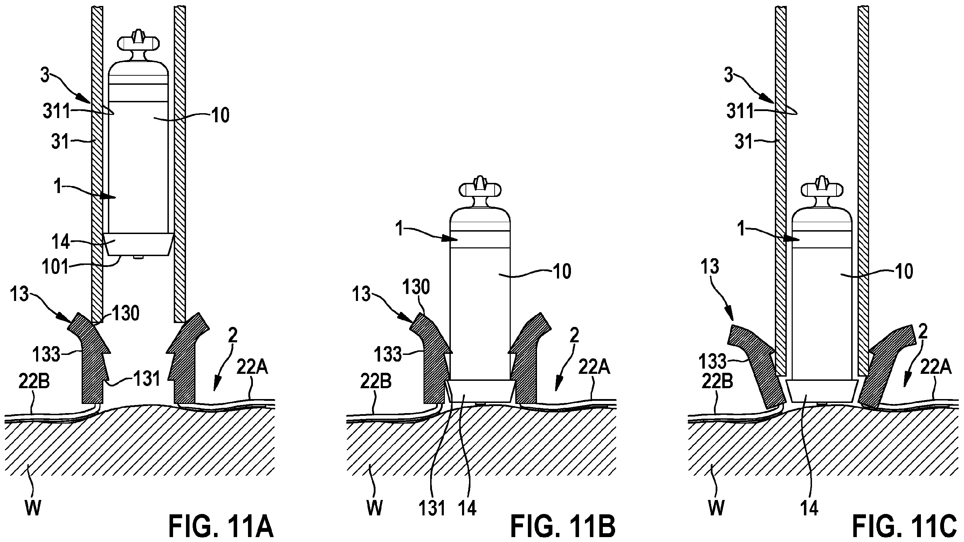

Referring now to Figs. 11A to 11C, for placing the implantable medical device 1 on the anchoring device 2 of Fig. 9, the implantable medical device 1 is delivered towards the anchoring device 2 using a delivery device 3, the anchoring device 2 already being implanted and positioned at a location of interest. For attaching the implantable medical device 1 on the anchoring device 2, the implantable medical device 1 is approached towards the docking mechanism 13 of the anchoring device 2 by means of a catheter 31 of the delivery device 3, and the implantable medical device 1 is pushed out of the catheter 31 to engage with the receptacle 130 of the housing portion 133 of the anchoring device 2, as shown in the transition of Figs. 11A to Fig. 11B.

In an attached state, shown in Fig. 11B, a locking element 14 of the implantable medical device 1 mechanically engages with the mechanical locking feature 131 of the anchoring device 2, such that a positive locking connection in between the implantable medical device 1 and the anchoring device 2 is established.

The anchoring device 2 may be shaped to allow for a disconnection of the implantable medical device 1 in order to for example replace the implantable negative device 1. For this, as illustrated in Fig. 11C, the delivery device 3 may again be approached towards the implantable medical device 1 to receive the implantable medical device 1 in the receptacle 311 of the catheter 31, the catheter 31 for example acting onto the housing portion 133 such that the mechanical connection in between the locking element 14 and the mechanical locking feature 131 is released. The implantable medical device 1 hence can be removed from the anchoring device 2.

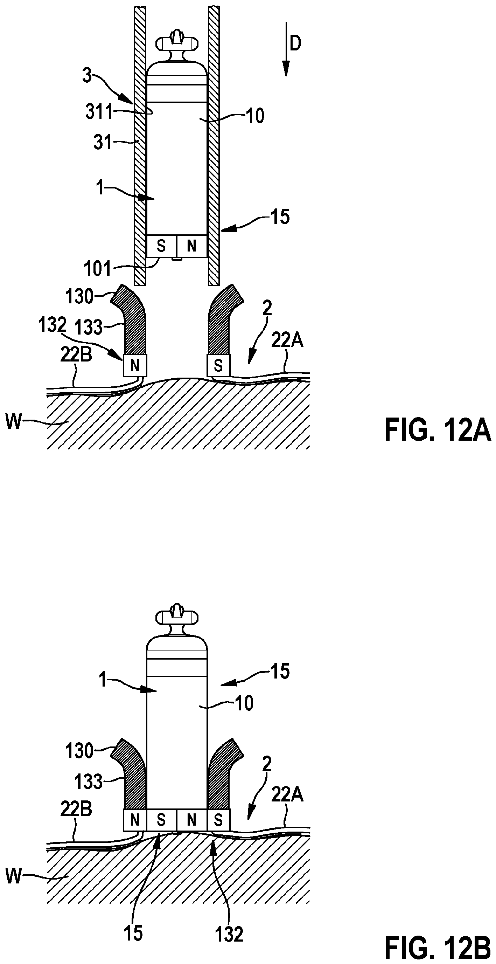

Referring now to Figs. 12A and 12B, illustrating the attachment of the implantable medical device 1 on the anchoring device 2 of Fig. 10, for placing the implantable medical device 1 on the anchoring device 2 the implantable medical device 1 is approached towards the receptacle 130 of the anchoring device 2 using a delivery device 3. By pushing the implantable medical device 1 out of the receptacle 311 of the catheter 31 the implantable medical device 1 is inserted into the receptacle 130 of the housing portion 133 and engages with the receptacle 130, a fixation of the implantable medical device 1 within the receptacle 130 being established by means of magnet elements 15, 132 of the anchoring device 2 and of the implantable medical device 1, as visible from Fig. 12B.

Also for the embodiment of Fig. 10 and Figs. 12A, 12B the implantable medical device 1 may be removed from the anchoring device 2 by using the delivery device 3 to disengage the implantable medical device 1 from the receptacle 130 of the anchoring device 2, similarly as shown in Fig. 11C. Alternatively, a specified pull force may be applied to the implantable medical device 1 for releasing the implantable medical device 1 from the docking mechanism 13 of the anchoring device 2.

In the embodiments of Figs. 9 to 12A, 12B the anchoring device 2 with respect to the abutment members 22, 22A, 22B may be configured as in the embodiment of Figs. 2 to 8A-8D, such that it shall be referred to the above in this respect.

The anchoring device 2 may provide for a safe and reliable anchoring on tissue. In embodiments in which the implantable medical device is releasably attached to the anchoring device an easy replacement of medical devices becomes possible.

In addition, the anchoring device 2 may comprise radiopaque elements or markers, particularly being integrally formed with the anchoring device 2, by which the anchoring device may be located or the spatial orientation thereof may be determined. Such radiopaque markers or element may particularly facilitate the deployment of the anchoring device 2 and the implantable medical device 1, respectively.

### LIST OF REFERENCE NUMERALS

- 1: Leadless pacemaker device
- 10: Housing
- 101: Housing portion (distal end)
- 102: Proximal end
- 11: Electrode
- 12: Coupling device
- 13: Docking mechanism
- 130: Receptacle
- 131: Locking feature
- 132: Magnet arrangement
- 14: Locking element
- 15: Magnet arrangement
- 2: Anchoring device
- 20: Strut
- 200,201: End
- 21: Sheet arrangement
- 22, 22A, 22B: Abutment member
- 23: Fixation arrangement
- 230: Engaging elements
- 231: Glue arrangement
- 232: Liner
- 3: Delivery device
- 30: Assembly tube
- 31: Catheter
- 310: Catheter distal end
- 311: Receptacle
- A: Longitudinal axis
- B: Assembly direction
- C: Removal direction
- D: Placement direction (distal direction)
- L: Length of extension
- LA: Left atrium
- LV: Left ventricle
- P: Proximal direction
- RA: Right atrium
- RV: Right ventricle
- W: Intra-cardiac wall

## Claims

1. An anchoring device (2) for anchoring an implantable medical device (1) on tissue, comprising:
a housing portion (101; 133);
at least one elastically deformable abutment member (22, 22A, 22B) configured to abut with tissue and extending from said housing portion (101; 133) along a length of extension (L); and
a fixation arrangement (23) arranged on the at least one abutment member (22, 22A, 22B) to fix the at least on abutment member (22, 22A, 22B) on tissue over at least a portion of said length of extension (L).

2. The anchoring device (2) of claim 1, wherein the at least one abutment member (22, 22A, 22B) is connected at a first end (200) to said housing portion (101; 133) and comprises a second, free end (201) remote from the housing portion (101; 133), the at least one abutment member (22, 22A, 22B) extending over said length of extension (L) between the first end (200) and the second, free end (201).

3. The anchoring device (2) of claim 1 or 2, wherein the fixation arrangement (23) comprises a multiplicity of engaging elements (230) distributed on the at least one abutment member (22, 22A, 22B) across at least a portion of said length of extension (L), the engaging elements (230) being configured to engage with tissue.

4. The anchoring device (2) of claim 1 or 2, wherein the fixation arrangement (23) comprises a glue arrangement (231) distributed on the at least one abutment member (22, 22A, 22B) across at least a portion of said length of extension (L), the glue arrangement (231) being configured to establish a gluing connection with tissue.

5. The anchoring device (2) of one of the preceding claims, wherein said at least one abutment member (22, 22A, 22B) comprises at least one elastically deformable strut (20).

6. The anchoring device (2) of one of the preceding claims, wherein said at least one abutment member (22, 22A, 22B) comprises a multiplicity of struts (20) arranged on the housing portion (101; 133), the housing portion (101; 133 being configured to be placed on tissue along a placement direction (D), wherein the struts (20) are circumferentially distributed about the placement direction (D).

7. The anchoring device (2) of one of the preceding claims, wherein said at least one abutment member (22, 22A, 22B) comprises a flexible sheet arrangement (21) for abutting with tissue.

8. The anchoring device (2) of claim 7, wherein the flexible sheet arrangement (21) extends in between at least two struts (20) connected to said housing portion (101; 133).

9. The anchoring device (2) of one of claims 1 to 8, wherein said housing portion (101; 133) is formed by a distal end of a housing (10) of the implantable medical device (1).

10. The anchoring device (2) of one of claims 1 to 8, comprising a docking mechanism (13) for attaching the implantable medical device (1) to the anchoring device (2), wherein the housing portion (101; 133) is formed by the docking mechanism (13).

11. The anchoring device (2) of claim 10, wherein said docking mechanism (13) comprises a magnet arrangement (132) configured to magnetically interact with the implantable medical device (1) to connect the implantable medical device (1) to the anchoring device (2).

12. The anchoring device (2) of claim 10, wherein said docking mechanism (13) comprises
- a mechanical locking feature (131) and/or
- an adhesive
for establishing a mechanical connection in between the anchoring device (2) and the implantable medical device (1).

13. A delivery arrangement for implanting said anchoring device (2) in a patient, comprising a delivery device (3) and an anchoring device (2) of one of the preceding claims, the delivery device (3) being configured to place the anchoring device (2) on tissue in a placement direction (D), wherein the delivery device (3) comprises a receptacle (311) for receiving the anchoring device (2) in a delivery state, wherein in the delivery state the at least one abutment member (22, 22A, 22B) is elastically deformed to extend from the housing portion (101; 133) in a direction opposite the placement direction (D).

14. A method for anchoring an implantable medical device (1) on tissue using an anchoring device (2), the method comprising:
placing a housing portion (101; 133) of the anchoring device (2) on tissue;
causing at least one elastically deformable abutment member (22, 22A, 22B) extending from said housing portion (101; 133) along a length of extension (L) to abut with tissue; and
fixing the at least on abutment member (22, 22A, 22B) on tissue over at least a portion of said length of extension (L) using a fixation arrangement (23) arranged on the at least one abutment member (22, 22A, 22B).

15. The method of claim 14, wherein said placing includes: placing the anchoring device (2) on tissue using a delivery device (3), the anchoring device (2) in a delivery state being received in a receptacle (311) of the delivery device (2), wherein in the delivery state the at least one abutment member (22, 22A, 22B) is elastically deformed to extend from the housing portion (101; 133) in a direction opposite the placement direction (D); and wherein said causing includes: exiting the anchoring device (2) from the delivery device (3) such that the at least one abutment member (22, 22A, 22B) elastically folds out from the delivery state to abut with tissue.
